# EUROPEAN PATENT SPECIFICATION

(11) **EP 4 501 397 B1**
(45) Date of publication and mention of the grant of the patent: **14.10.2026**
(21) Application number: 23777559.8
(22) Date of filing: 03.01.2023
(51) Int. Cl.: A61N 1/36, A61N 1/372, A61N 1/02, H02J 50/12, H02J 50/80, A61N 1/378

(54) **CAPACITIVE COUPLING ENERGY TRANSMISSION NERVE ELECTROSTIMULATION SYSTEM, IN-VIVO NERVE ELECTROSTIMULATOR THEREOF, AND IN-VITRO ENERGY CONTROLLER THEREOF**
NERVENELEKTROSTIMULATIONSSYSTEM MIT KAPAZITIVER KOPPLUNGSENERGIEÜBERTRAGUNG, IN-VIVO-NERVENELEKTROSTIMULATOR UND IN-VITRO-ENERGIESTEUERUNG DAFÜR
SYSTÈME D'ÉLECTROSTIMULATION DE NERF À TRANSMISSION D'ÉNERGIE PAR COUPLAGE CAPACITIF, ÉLECTROSTIMULATEUR DE NERF IN VIVO ASSOCIÉ, ET DISPOSITIF DE COMMANDE D'ÉNERGIE IN VITRO ASSOCIÉ

(30) Priority: 28.03.2022 CN 202210315229
(43) Date of publication of application: 05.02.2025
(73) Proprietor: BEIJING LEADING INNOVATION MEDICAL VALLEY CO., LTD, Beijing 100162 (CN)
(72) Inventor: XU, Tianrui, Beijing 100162 (CN)
(74) Representative: EP&C
(86) International application number: PCT/CN2023/070095
(87) International publication number: WO 2023/185180

(56) References cited:
- WO-A1-2021/124303
- CN-A- 109 603 007
- CN-A- 111 167 012
- CN-A- 112 972 895
- CN-A- 113 274 640
- CN-A- 114 768 092
- CN-U- 217 697 661
- US-A1- 2019 269 399
- US-A1- 2020 376 275
- US-B1- 6 415 184

## Description

### Technical Field

The present disclosure relates to a nerve electrostimulation system of transcutaneous non-contact energy transmission.

### Background Art

Unlike implantable battery power supply, the transcutaneous energy transmission system (TETS) is to transmit an extraneous energy source through the human skin to an in-vivo implanted device by way of magnetic fields and electromagnetic induction. According to different working frequencies and power levels, the transcutaneous energy transmission method currently used in human implantable devices can be divided into two categories: radio frequency energy transmission and non-contact transformer based TETS.

In-vitro radio frequency power supply technology is currently widely used for energy and data transmission in human implanted devices, which provides electrical energy from in vitro to in vivo, or directly supply power to in-vivo electrical circuits, or charge in-vivo rechargeable batteries through electromagnetic induction of radio frequency and magnetic field coupling. In recent years, due to increasing complexity of implanted electronic systems, the power consumption of in-vivo electronic systems has also been increasing. Due to limitations of its own topology, the radio frequency power supply technology cannot achieve the electrical energy supply for high-power implanted devices such as artificial hearts.

For example, Chinese patent CN103796715B discloses a neural stimulator system that uses an in-vitro controller to directly transmit electrical energy to an in-vivo implanted part through an RF antenna. Due to the aforementioned limitations, this system has a short transmission distance (<6cm), and its transmission power is limited by the battery capacity of the in-vitro controller. Patent application publication WO2021124303A1 discloses an implantable device implantable via a vessel in the body, the implantable device comprising external conductive features and being configured to receive power via the conductive features from an electromotive force applied to body tissue.

To solve the above problems, the transcutaneous energy transmission using non-contact transformers continues to increase. Similar to the principle of radio frequency energy transmission, both use the principle of electromagnetic induction to achieve electromagnetic conversion, magnetoelectric conversion, and non-contact transmission of energy. The difference is that this new type of induced electrical energy transmission utilizes a non-contact transformer having large air gaps between the primary and secondary sides as the key link for energy transmission, in which the secondary side of the transformer is implanted inside the human body, and the primary side of the transformer located outside the body facing to the secondary side, transmitting electrical energy through skin through electromagnetic induction in the form of a magnetic field to the in-vivo implanted device. Compared to the RF electrical energy transmission mentioned above, this system has a relatively low operation frequency. Due to large leakage inductance of the separated transformer, the energy transmission efficiency of the system is adversely affected.

The information disclosed in the background section of the present disclosure is only intended to increase an understanding of the overall background of the present disclosure,
and should not be regarded as acknowledging or implying in any form that the information constitutes the prior art that is already well-known to those skilled in the art.

### Summary of the Invention

The present invention is defined by the appended claims. Aspects, embodiments and examples described hereinafter are only of exemplary nature and are presented for better understanding the present invention. The disclosure aims to propose an implantable nerve electrostimulator system based on capacitive coupling for energy transmission. Compared to an implantable nerve electrostimulator system that transmits energy through radio frequency or transformers, the capacitive coupling electrical energy transmission of the present disclosure can transmit higher electrical power.

The present disclosure provides an in-vivo nerve electrostimulator comprising: at least one stimulator coupling capacitor pole for coupling with an energy controller coupling capacitor pole of an in-vitro energy controller, thereby receiving electrical energy from the in-vitro energy controller and achieving information exchange; a stimulator compensation resonant network connected to the stimulator coupling capacitor pole to compensate for reactive power of the received electrical energy; a rectification circuit connected to the stimulator compensation resonant network and converting alternating current energy that has been compensated by a compensation couple into direct current energy; a filter capacitor connected to the rectification circuit to perform direct current filtering on the rectified direct current energy; a main control chip that controls operation of the in-vivo nerve electrostimulator, connected to the rectification circuit and the filter capacitor for receiving power supply therefrom; a stimulator harmonic communication module connected between the stimulator coupling capacitor pole and the main control chip for modulating and demodulating communication information; a plurality of sets of stimulation electrodes and corresponding balance capacitors, wherein each balance capacitor is connected between the main control chip and a corresponding stimulation electrode, receives stimulation pulses from the main control chip and applies them to the corresponding stimulation electrode, and achieves charge balance.

In the above in-vivo nerve electrostimulator, preferably it comprises two stimulator coupling capacitor poles, wherein the stimulator compensation resonant network is formed as follow: one of the two stimulator coupling capacitor poles is connected to the rectification circuit through a stimulator compensation inductor, and the other one of the two stimulator coupling capacitor poles is directly connected to the rectification circuit.

In the above in-vivo nerve electrostimulator, preferably the stimulator harmonic communication module comprises a filter circuit sensitive to A-th harmonic, thereby extracting information from the A-th harmonic of the received signal and transmitting the information to the main control chip. Further preferably, the stimulator harmonic communication module also comprises a filter circuit sensitive to B-th harmonic, thereby extracting the B-th harmonic from the received signal, and the main control chip controls the stimulator harmonic communication module to modulate impedance of the B-th harmonic, and the impedance modulated data is transmitted to the in-vitro energy controller by the stimulator coupling capacitor pole. Optionally, wherein B may be equal to A.

The present disclosure also provides an in-vitro energy controller for transmitting electrical energy to and communicating with an in-vivo nerve electrostimulator, comprising: a battery module for supplying power to the in-vitro energy controller; an energy controller control unit for controlling the operation of the in-vitro energy controller; an upper computer communication module connected to the energy controller control unit which communicates with a program controller through the upper computer communication module; a storage module for storing electrical stimulation signals and connected to the energy controller control unit; an inverter module connected to the battery module and the energy controller control unit for converting direct current supplied by the battery module into alternating current; an energy controller compensation resonant network connected to the inverter module to compensate for reactive power of the system, at least one energy controller coupling capacitor pole connected to an output end of the energy controller compensation resonant network; an energy controller harmonic communication module powered by the battery module and bridged between the energy controller control unit and the energy controller coupling capacitor pole for modulating and demodulating communication information; and a button and display module comprising a button for inputting an operation instruction and a display screen for displaying input content and information about operation of the in-vivo nerve electrostimulator.

In the above in-vitro energy controller, preferably it comprises two energy controller coupling capacitor poles, wherein the energy controller compensation resonant network forms a serial inductance compensation network as follow: one of the two energy controller coupling capacitor poles is connected to the inverter module through a first energy controller compensation inductor, and the other one of the two energy controller coupling capacitor poles is directly connected to the inverter module .

In the above in-vitro energy controller, preferably it comprises two energy controller coupling capacitor poles, wherein the energy controller compensation resonant network forms an LCL compensation network topology as follow: one of the two energy controller coupling capacitor poles is connected in series to the inverter module through one first energy controller compensation inductor and another first energy controller compensation inductor; the other one of the two energy controller coupling capacitor poles is directly connected to the inverter module to form a connection point, which is connected to a common connection end of the one first energy controller compensation inductor and the another first energy controller compensation inductor through a first energy controller compensation capacitor.

In the above in-vitro energy controller, preferably it comprises two energy controller coupling capacitor poles, wherein the energy controller compensation resonant network forms a compensation resonant network structure having a relay coil as follow: comprising a primary winding having one end connected to a first output end of the inverter module and the other end connected to a second output end of the inverter module through a second energy controller compensation capacitor; and a secondary winding having one end connected to one of the energy controller coupling capacitor poles and the other end connected to the other one of the energy controller coupling capacitor poles.

In the above in-vitro energy controller, preferably the energy controller control unit controls the energy controller harmonic communication module to generate an A-th harmonic equivalent to A times of the output frequency of the inverter module, and modulates information into the A-th harmonic. Further preferably, the energy controller control unit controls the energy controller harmonic communication module to generate a B-th harmonic equivalent to B times of the output frequency of the inverter module, and transmits the harmonic to the pole. Optionally, wherein B can be equal to A.

In the above in-vitro energy controller, preferably the upper computer communication module is a Bluetooth module or a WiFi module.

The present disclosure also provides a nerve electrostimulation system using capacitive coupling energy transmission comprising the in-vivo nerve electrostimulator and the in-vitro energy controller.

In the above nerve electrostimulation system using capacitive coupling energy transmission, preferably it further comprises a program controller which has a program controller communication module for communicating with the in-vitro energy controller and an upper computer control APP for achieving human-machine interaction control to the in-vitro energy controller and the in-vivo nerve electrostimulator.

In the above nerve electrostimulation system using capacitive coupling energy transmission, preferably the program controller communication module is a Bluetooth module or a WiFi module.

According to the implantable nerve electrostimulator system of the present disclosure,
due to use of capacitive coupling for electric energy transmission, it can transmit greater electrical power, thereby expanding its applicability.

The methods and devices of the present disclosure have other characteristics and
advantages that will be apparent from the accompanying drawings and subsequent specific embodiments incorporated herein, or will be described in detail in the accompanying drawings and subsequent specific embodiments, which together serve to explain the specific principles of the present disclosure.

### Brief Description of Drawings

Figure 1 shows a functional block diagram of a nerve electrostimulator system.
Figure 2 shows a functional block diagram of a program controller.
Figure 3 shows a functional block diagram of an in-vitro energy controller.
Figure 4 shows a functional block diagram of an in-vivo nerve electrostimulator.
Figure 5 shows a block diagram of a main control chip of an in-vivo nerve electrostimulator.
Figure 6 shows a schematic diagram of implementation of a capacitive coupling capacitor pole.
Figure 7 shows a first embodiment of a compensation resonant network.
Figure 8 shows a second embodiment of a compensation resonant network.
Figure 9 shows a third embodiment of a compensation resonant network.
Figure 10 shows a power on and authentication process of a main control chip.
Figure 11 shows a power on and reset waveform of a main control chip.
Figure 12 shows an impedance measurement flowchart.
Figure 13 shows a flowchart of a writing process of electrostimulation parameters.

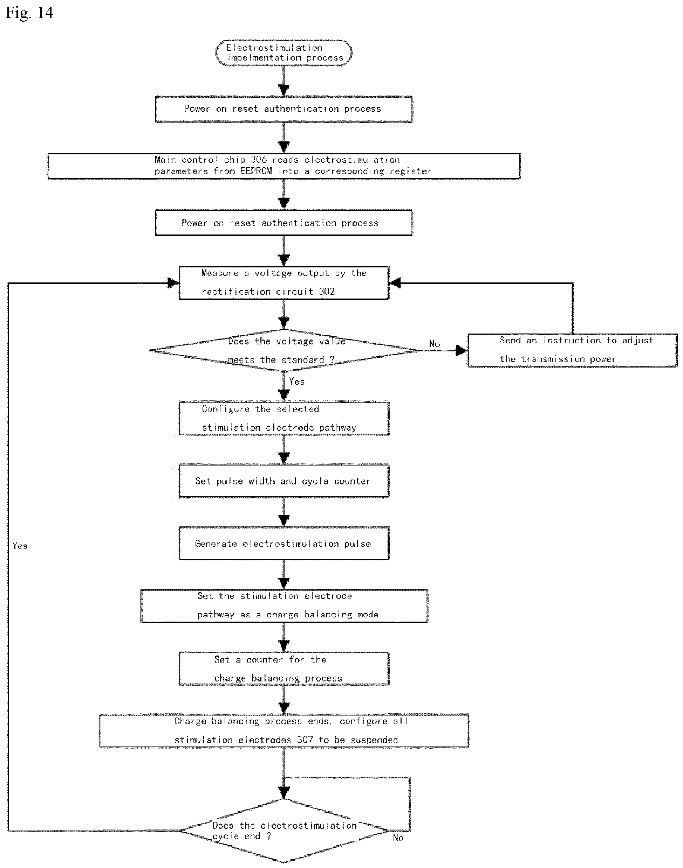

Figure 14 shows a flowchart of implementation of electrostimulation.
Figure 15 shows a first type of electrostimulation waveform I.
Figure 16 shows a second type of electrostimulation waveform II.
Figure 17 shows a third type of electrostimulation waveform III.
Figure 18 shows a fourth type of electrostimulation waveform IV.
Figure 19 shows a fifth type of electrostimulation waveform V.
Figure 20 shows a flowchart of manual adjustment mode.
Figure 21 shows a flowchart of debugging a new device.

It should be understood that the accompanying drawings do not necessarily have to be drawn to scale, as they illustrate various features of the basic principles of the present disclosure that have been simplified to some extent. The specific design features of the present disclosure disclosed herein, including specific dimensions, orientation, positioning, and appearance, will be partially determined by the application and usage environment for the specific purpose.

In these drawings, in multiple figures running through the drawings, the reference numerals refer to the same or equivalent parts of the present disclosure.

List of reference numerals:
01 program controller
02 in-vitro energy controller
201 energy controller control unit
202 Bluetooth module
203 storage module
204 inverter module
205 energy controller harmonic communication module
206 battery module
207 energy controller coupling capacitor pole
208 energy controller compensation resonant network
209 button and display module
210 WiFi module
03 in-vivo nerve electrostimulator
301 stimulator coupling capacitor pole
302 rectification circuit
303 stimulator harmonic communication module
304 filter capacitor
305 stimulator compensation resonant network
306 main control chip
307 stimulation electrode
308 balance capacitor.

### Detailed Description of Embodiments

Now, specific reference will be made to the various embodiments of the present disclosure, and examples of these embodiments are shown in the accompanying drawings and the following description.

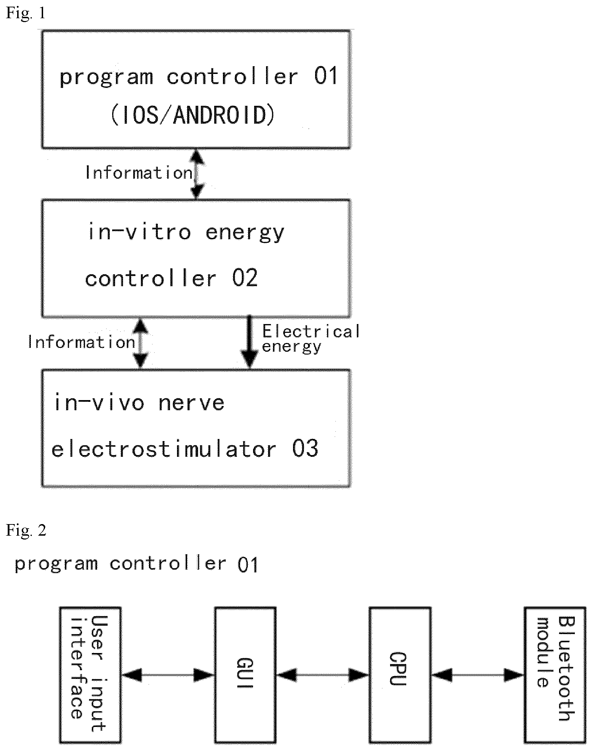

As shown in Figure 1, the nerve electrostimulation system using capacitive coupling energy transmission of the present disclosure comprises three parts: a program controller 01 installed with a hardware carrier of an upper computer APP (for example, a hardware carried with an IOS system, such as an iPad, etc.), an in-vitro energy controller 02, and an in-vivo nerve electrostimulator 03. The program controller 01 can communicate with the in-vitro energy controller 02 through a Bluetooth module or an optional WiFi module. The in-vitro energy controller 02 communicates with the in-vivo nerve electrostimulator 03 through a harmonic communication module or a Bluetooth module. The in-vivo nerve electrostimulator 03 obtains electrical energy from the in-vitro energy controller 02 in the form of capacitive coupling.

As a main information exchange platform, the program controller 01 and its APP can achieve all functions such as controlling, debugging programs, querying patient related information, and configuring the in-vivo nerve electrostimulator 03. Doctors can manage patient information, set treatment schemes, adjust parameter configurations, and view relevant data recorded during the electrostimulation treatment process through the program controller 01.

The program controller 01 shown in Figure 2 is provided with a Bluetooth module. At this point, the program controller 01 communicates with the in-vitro energy controller 02 through Bluetooth protocol.

In addition, a WiFi module can be integrated into the in-vitro energy controller, which enables doctors to remotely view patient data and adjust treatment schemes. The WiFi module can be an optional accessory, which is designated by the patient to be configured or not.

The in-vitro energy controller 02 is a relay part that enables data transmission, control, debugging, and other functions between the program controller 01 and the in-vivo nerve electrostimulator 03; meanwhile, it is also a main part that supplies power for in-vivo devices.

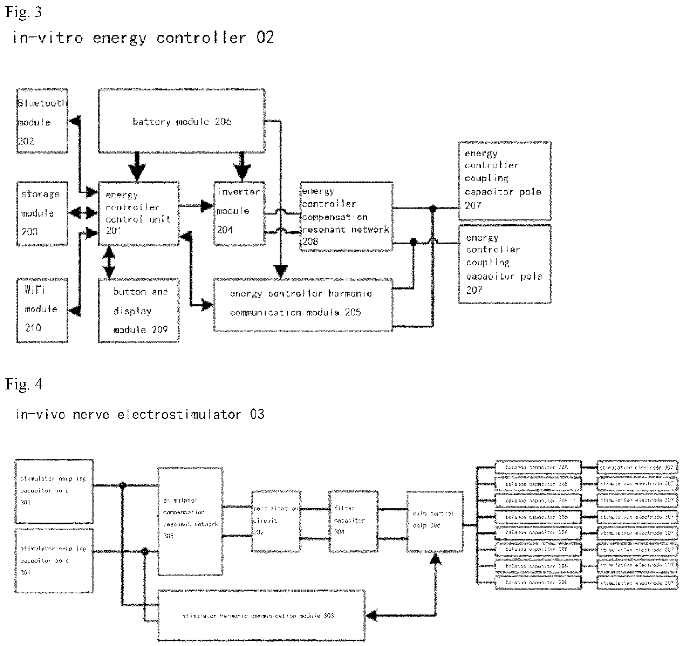

As shown in Figure 3, the in-vitro energy controller 02 includes an energy controller control unit 201, a Bluetooth module 202, a storage module 203, an inverter module 204, an energy controller harmonic communication module 205, a battery module 206, and at least one energy controller coupling capacitor pole 207, an energy controller compensation resonant network 208, and a button and display module 209. A WiFi module 210 can also be optionally equipped, as shown in Figure 3.

The in-vitro energy controller 02 communicates with the program controller 01 through the Bluetooth module 202, and configures internal register data according to the information obtained from the program controller 01. All parameters of the electrostimulation signal to be generated are stored in the storage module 203 (non-volatile memory can be used), and can be reliably stored for a long time.

At the same time, the in-vitro energy controller 02 converts direct current of a battery pack into high-frequency alternating current through the inverter module 204, which is transmitted to the energy controller coupling capacitor pole 207 after being adjusted by the energy controller compensation resonant network 208 in order to supply power to the in-vivo nerve electrostimulator 03. The main control chip uses the second or higher-order harmonics of high-frequency alternating current generated by the inverter module 204 for information modulation and demodulation, and communicates with the in-vivo energy controller through the energy controller harmonic communication module 205.

That is, in the in-vitro energy controller, the inverter circuit converts direct current into alternating current, which is composed of harmonics of different orders, with the fundamental wave having the highest energy and used for electrical energy transmission. In addition to the fundamental wave, the filter circuit also extracts the A-th and B-th harmonics. The A-th harmonic is used to transmit information data to the inside of body, while the B-th harmonic is used to transmit data from the inside to the outside of body.

The in-vivo nerve electrostimulator 03 includes at least one stimulator coupling capacitor pole 301 for receiving electrical energy of the in-vitro energy controller, as well as a rectification circuit 302, a stimulator harmonic communication module 303, a filter capacitor 304, a stimulator compensation resonant network 305 and other discrete elements connected thereto. In addition, it also includes a main control chip 306 and several sets of stimulation electrodes 307 and balance capacitors 308 to achieve electrostimulation function, as shown in Figure 4.

In addition, due to the capacitive coupling way being able to transmit more electrical energy, the in-vivo nerve electrostimulator 03 can also be equipped with a Bluetooth module. In this case, the in-vitro energy controller can also communicate in real-time bilaterally with the in-vivo nerve electrostimulator 03 through Bluetooth.

The stimulator coupling capacitor pole 301 obtains electrical energy from the energy controller coupling capacitor pole 207 of the in-vitro energy controller in the way of capacitive coupling, and compensates for reactive power through the stimulator compensation resonant network 305. The alternating current energy is converted into direct current energy through a rectification circuit 302, which is filtered through the filter capacitor 304 for direct current filtering. This capacitor can also store a small amount of electrical energy. The direct current energy having been rectified and filtered is supplied to the main control chip 306 for use. In addition, the stimulator harmonic communication module 303 is connected to the stimulator capacitor pole 301 to achieve functions of modulating and demodulating the harmonic signals. And the data is exchanged with the main control chip.

The stimulation electrode 307 is a main executing component of nerve electrostimulation, which implements electric pulse stimulation on the nerves. The function of the balance capacitor 308 is to ensure that the static charge of each electrode 307 is zero during the implementation of nerve electrostimulation, achieving charge balance.

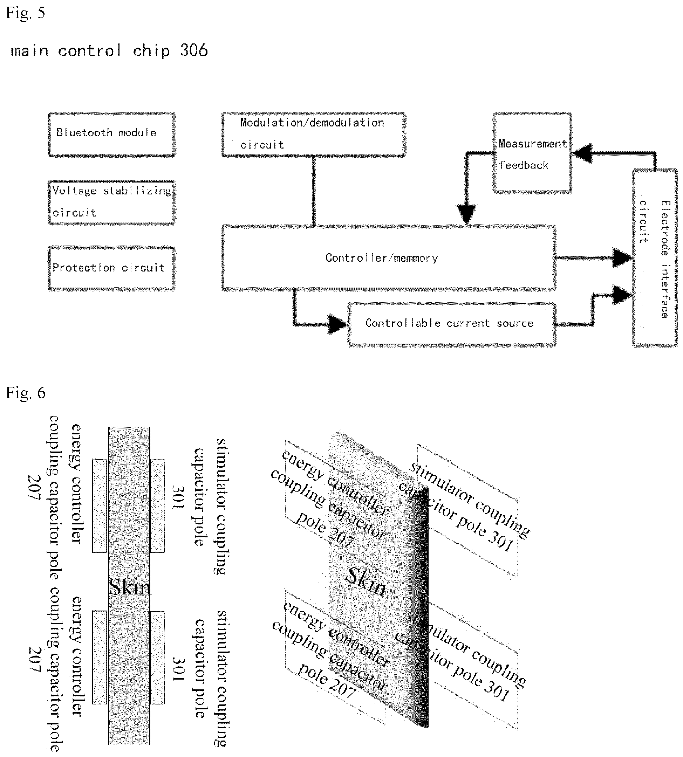

The main control chip 306 can achieve functions such as configuration information storage, data calculation, and controlling generation of electrostimulation pulses, which is a main control component for achieving in-vivo electrostimulation functions. The main control chip 306 can also integrate with a Bluetooth module, which replaces the harmonic communication module. The internal block diagram of the main control chip 306 is shown in Figure 5.

The main control chip 306 is internally configured with a controller, a memory, a controllable current source, an electrode interface circuit, a measurement feedback circuit, and a modulation and demodulation circuit. In addition, a voltage stabilizing circuit and an overcurrent protection circuit are also configured at the power supply end to ensure that the power supply voltage does not exceed the warning voltage value, thereby protecting the main control chip 306. The memory can use a non-volatile memory to achieve power-off storage and to reliably store the relevant set parameters of electrostimulation pulses for a long time. The chip can be optionally equipped with a Bluetooth module internally to support the Bluetooth communication protocol.

The main control chip 306 controls the controllable current source circuit through a controller to generate the required current pulse stimulation signal, and transmits the pulse to the designated electrode through an electrode interface circuit.

Figure 6 is a schematic diagram of implementation of the energy controller coupling capacitor pole 207 and the stimulator coupling capacitor pole 301. As shown in Figure 6, when in use, the energy controller coupling capacitor pole 207 of the in-vitro energy controller 02 and the stimulator coupling capacitor pole 301 of the in-vivo nerve electrostimulator 03 are respectively provided on the outer and inner sides of the patient's skin.

The following provides a detailed explanation of operation of the nerve electrostimulation system using capacitive coupling energy transmission of the present disclosure.

### Electrical Energy Transmission Procedure

The electrical energy transmission is mainly controlled by the in-vitro energy controller 02, and the battery module 206 in the in-vitro energy controller 02 is a main electrical energy storage component. The energy controller control unit 201 controls the inverter module 204 to invert direct current energy provided by the battery module 206 into high-frequency alternating current, with a frequency ranging from 1MHz to 40MHz. Due to the capacitive coupling between the energy controller coupling capacitor pole 207 and the stimulator coupling capacitor pole 301, there is a relatively large equivalent serial capacitance. Therefore, in order to compensate for the reactive power of the system, an energy controller compensation resonant network 208 and a stimulator compensation resonant network 305 are added. For the stimulator compensation resonant network 305 of the in-vivo implanted part, simple serial inductance compensation or no compensation can be used due to the need of considering factors such as magnetic resonance compatibility and volume.

However, there may be a plurality of optional implementation schemes for the energy controller compensation resonant network 208 of the in-vitro energy controller portion.

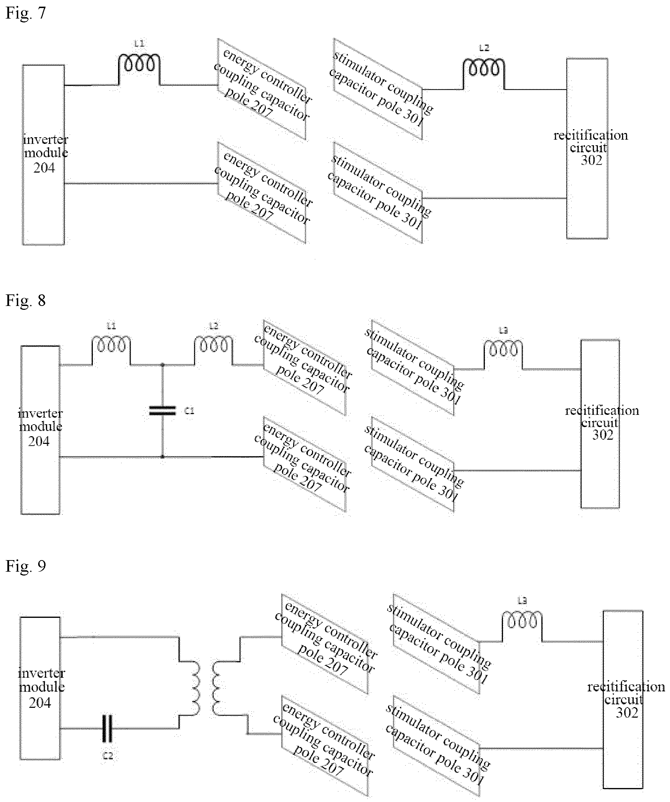

Scheme 1 is the simplest serial inductance compensation, as shown in Figure 7.

Scheme 2 is the LCL compensation network topology, as shown in Figure 8. Scheme 2 can effectively compensate for reactive power while also transforming the impedance characteristics of the system to improve the problem of resonance frequency drift of the system.

Scheme 3 is a scheme of a compensation resonant network with a relay coil, in which the energy controller coupling capacitor pole 207 generates strong resonance with the secondary winding of the relay coil, which can increase the performance of the maximum transmission power of the system.

After passing through the equivalent capacitance between the energy controller coupling capacitor pole 207 and the stimulator coupling capacitor pole 301, electrical energy can be transmitted to the rectification circuit 302 of the in-vivo nerve electrostimulator 03, which converts the high-frequency alternating current energy into direct current energy for use by the in-vivo nerve electrostimulator 03.

### Communication Implementation Procedure

For an optional scheme in which the main control chip 306 of the in-vivo nerve electrostimulator 03 integrates a Bluetooth module, communication can be achieved through the Bluetooth transmission protocol, so it will not be elaborated here. The following introduces an implementation scheme using a harmonic communication module.

In the scheme of using harmonics for communication, when the in-vitro energy controller 02 transmits data to the in-vivo nerve electrostimulator 03, the energy controller control unit 201 controls the energy controller harmonic communication module 205 to generate an A-th harmonic equivalent to A times of the output frequency of the inverter module 204, and modulates the information into the A-th harmonic, which modulating may use an ASK way. The A-th harmonic enters the in-vivo nerve electrostimulator 03 through coupling of the stimulator coupling capacitor pole 301 and the energy controller coupling capacitor pole 207.

The stimulator harmonic communication module 303 of the in-vivo nerve electrostimulator 03 is configured with a filter circuit sensitive to the A-th harmonic. After being extracted by the filter circuit, the information is transmitted to the main control chip 306 for processing.

When the in-vitro energy controller 02 needs to read data of the in-vivo nerve electrostimulator 03, the in-vitro energy controller 02 controls the energy controller harmonic communication module 205 to generate the B-th harmonic, which is transmitted to the energy controller coupling capacitor pole 207.

The B-th harmonic can be transmitted to the in-vivo nerve electrostimulator 03 through the capacitive coupling between the energy controller coupling capacitor pole 207 and the stimulator coupling capacitor pole 301. The main control chip 306 controls the stimulator harmonic communication module 303 to modulate the impedance of the B-th harmonic, and transmits data to the in-vitro energy controller 02 in a manner of impedance modulation. The energy controller harmonic communication module 205 in the in-vitro energy controller 02 measures the equivalent impedance value of the system under the B-th harmonic, in order to obtain feedback data of the in-vivo nerve electrostimulator 03.

The A-th harmonic and B-th harmonic may have different harmonic orders or same harmonic order. They may be a fixed harmonic order or a real-time changing harmonic order. And they may be digital modulation or analog modulation.

### Power On Authentication Process of Main Control Chip

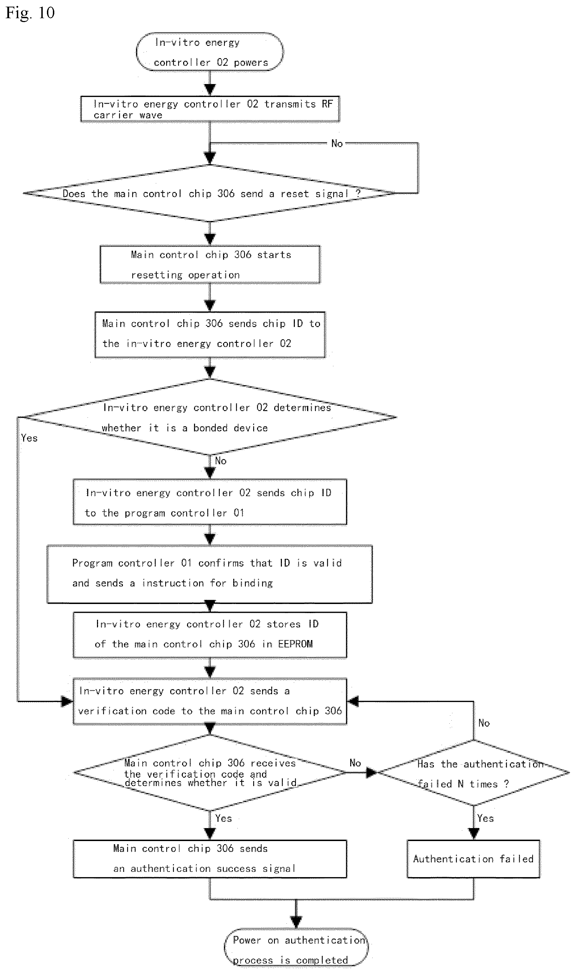

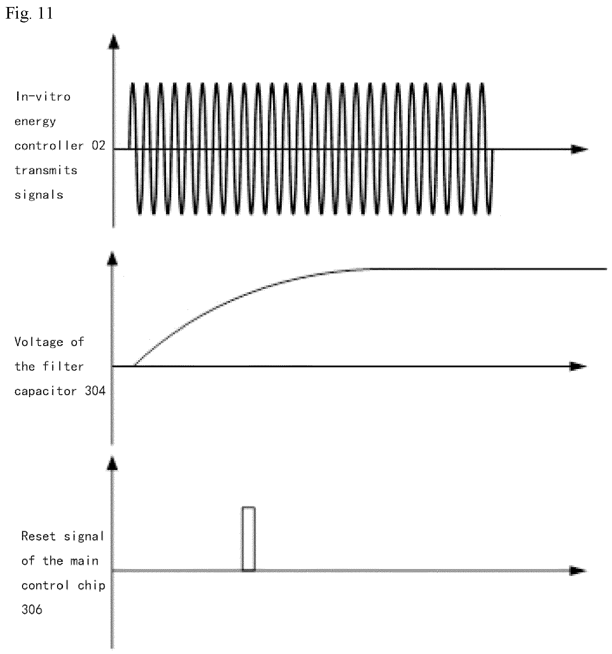

As shown in Figure 10, firstly, the in-vitro energy controller 02 performs energy transmission through the capacitive coupling between the energy controller coupling capacitor pole 207 and the stimulator coupling capacitor pole 301, and waits for the main control chip 306 in the in-vivo nerve electrostimulator 03 to be powered on and reset. After the internal circuit of the in-vivo nerve electrostimulator 03 reaches the specified voltage on the filter capacitor 304, a reset signal is generated, and the main control chip 306 starts working and feeds back on chip ID (i.e. chip identification) to outside. The power on and reset signals are shown in Figure 11.

When the in-vitro energy controller 02 receives the chip ID information for the first time, it transmits the information to the program controller 01 for confirmation, and records the chip ID in the EEROM memory to achieve device binding. After device binding, the program controller 01 is no longer required to participate in authentication again.

### Impedance Measurement Process

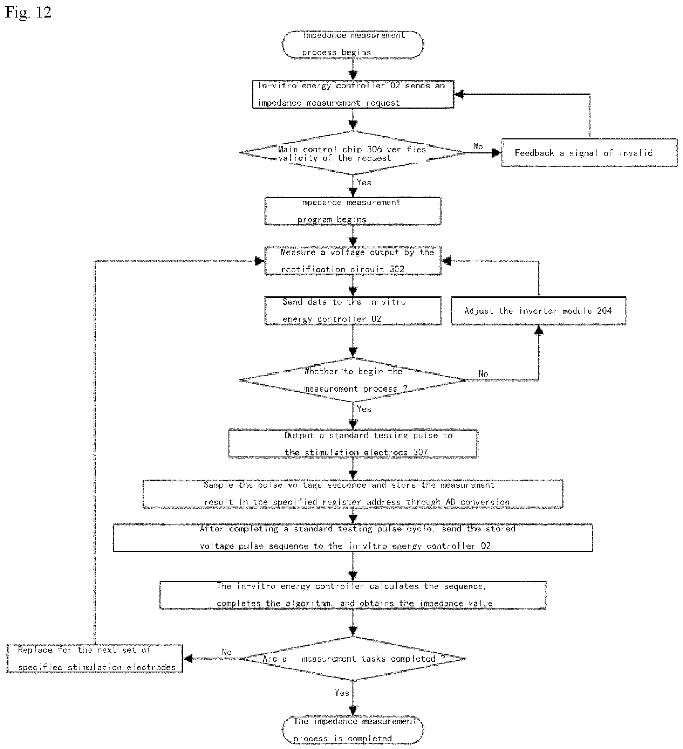

The in-vitro energy controller 02 makes a impedance measurement request to the in-vivo nerve electrostimulator 03 and sends a key. After receiving the request, the main control chip 306 of the in-vivo nerve electrostimulator 03 first verifies validity of the key, and then begins the impedance measurement program. The measurement procedure is shown in Figure 12.

The main control chip 306 firstly measures a voltage value output by the rectification circuit and sends this voltage value to the in-vitro energy controller 02. The in-vitro energy controller 02 determines whether to perform adjustment based on the voltage value (Figure 12 shows adjusting the inverter module), or whether to start the impedance measurement process. After receiving an instruction from the in-vitro energy controller to start the measurement process, the main control chip 306 controls loading of current pulses with standard amplitude and duration onto a certain electrode combination according to the standard testing program, and measures the voltage between an emitting electrode and a returning electrode. After AD conversion, the measured value is stored in the designated storage space inside the main control chip 306. After one electrostimulation pulse cycle, the measurement data is transmitted to the in-vitro energy controller 02. After the data is received and passes through verification, the in-vitro energy controller 02 transmits the data to the program controller 01 and transmits a confirmation information to the in-vivo nerve electrostimulator 03. After receiving the confirmation information, the in-vivo nerve electrostimulator 03 begins an impedance testing process for the next set of electrodes.

The so-called "electrode combination" refers to a set of electrodes of the same polarity, including an emitting electrode set (the electrode polarity in this set is positive), a returning electrode set (the electrode polarity in this set is negative), and a vacant electrode set (the electrode polarity in this set is 0). By distributing electrode polarity, various electrode combinations can be formed.

This cycle is repeated until all impedance measurement programs are completed. All data is finally summarized in the program controller 01 for storage and calculation, and all inter-electrode impedance that need to be measured are calculated.

### Electrostimulation Implementation Process

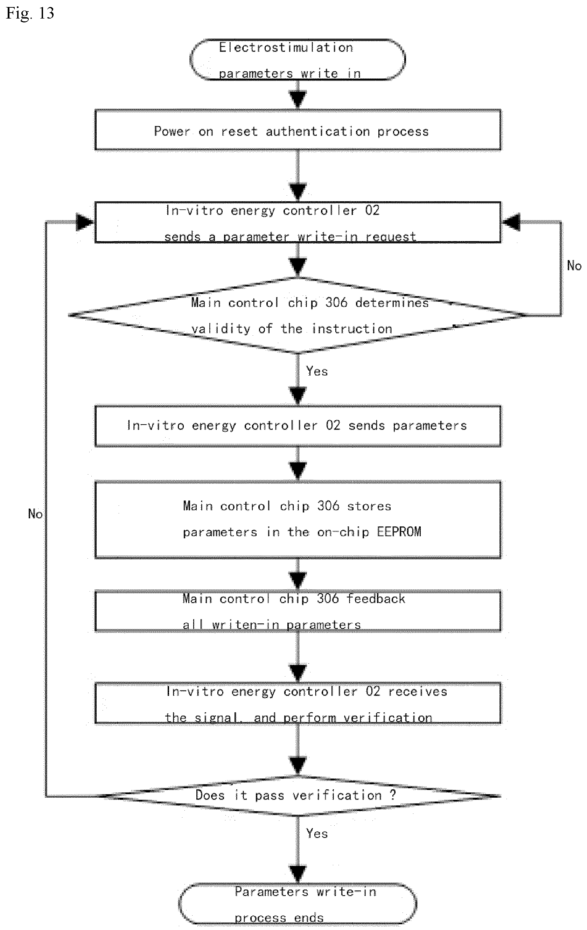

Figure 13 shows a writing process of electrostimulation parameters. Through this process, the program controller 01 including an upper computer control APP writes electrostimulation parameters into the in-vivo nerve electrostimulator 03 just through the in-vitro energy controller 02. All required parameters for electrostimulation will be stored in the memory of the in-vivo nerve electrostimulator 03 for a long time.

No active control is required from an external transmitter during the implementation of conventional electrostimulation. Detailed implementations are as follows.

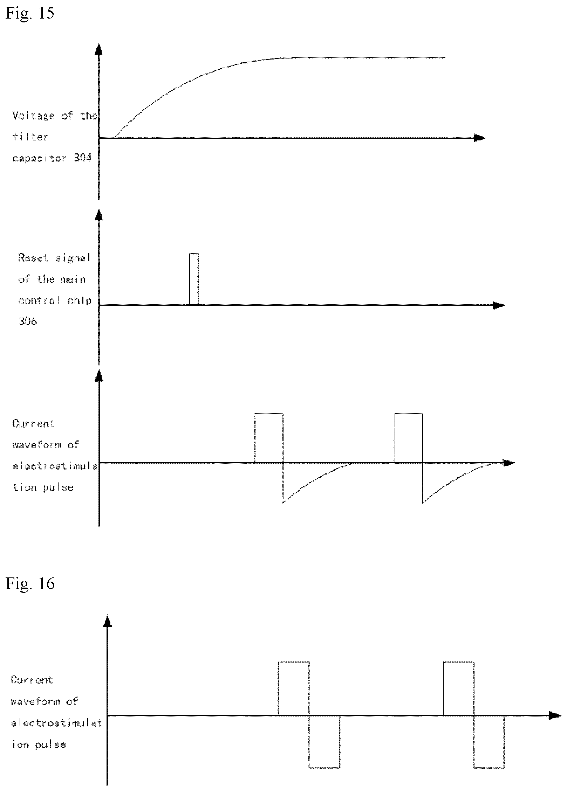

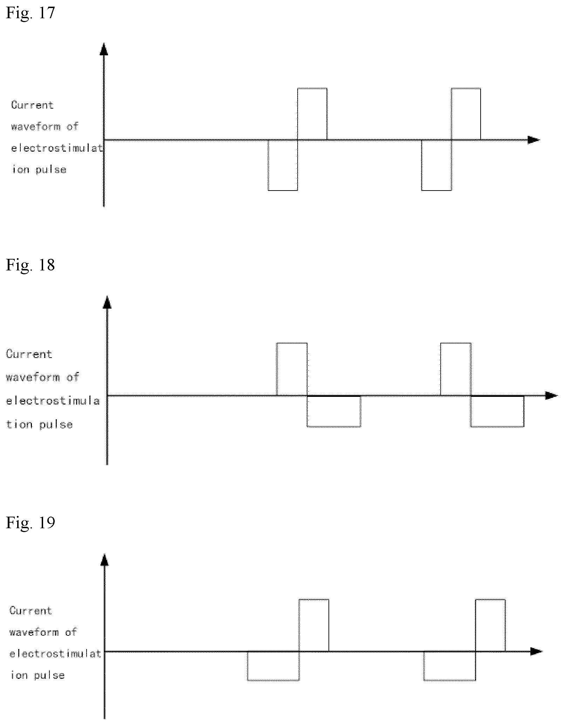

Firstly, the power on authentication process proceeds. After the power on authentication is completed, the in-vivo nerve electrostimulator 03 will generate a required current source pulse stimulation signals according to the stored electrostimulation parameters, and load the stimulation signal onto the corresponding electrodes. Then the actual stimulation current is measured and the measured value is stored in a corresponding position of the data memory of the chip. After implementation of one electrostimulation pulse, a charge balancing process is carried out to restore the net charge on the electrode to 0, which may be divided into two types: an active charge balancing process and a passive charge balancing process. The waveforms of the electrostimulation implemented may be waveforms as shown in Figures 15, 16, 17, 18, and 19, but are not limited to the above waveforms. Through internal control of the chip, as long as the net charge of the electrostimulation waveform at a single electrode end is zero, any shape of electrostimulation waveform may be output.

When the charge balancing process ends, waiting for the end of the entire pulse cycle, then implementation of a complete pulse stimulation process is finished.

The main control chip 306 confirms the required direct current voltage value based on feedback and compares it with the actual direct current rectification voltage value output by the rectification circuit 302.

When the rectification voltage value is insufficient, the main control chip 306 sends a request to the in-vitro energy controller 02 to increase the transmission power level. When the rectification voltage value is too high, the main control chip 306 sends a request to the in-vitro energy controller 02 to decrease the transmission power.

The in-vitro energy controller 02 adjusts the transmission power based on the received request.

### Patient Manual Adjustment Mode

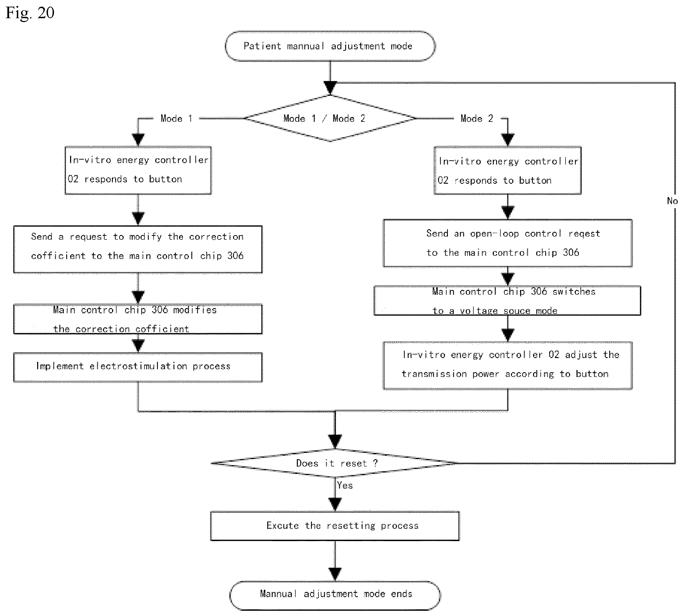

This system provides a patient manual adjustment mode, and a patient can switch to the manual adjustment mode at any time according to personal feeling. The manual adjustment mode has two implementation methods, as shown in Figure 20.

Implementation method 1: a correction coefficient type of manual adjustment.

In the implementation method 1, the patient manually adjusts a customized digital correction amount on the in-vitro energy controller 02. The in-vitro energy controller 02 sends the digital correction amount to the RAM of the in-vivo nerve electrostimulator 03. The in-vivo nerve electrostimulator 03 adds the correction amount to the originally stored electrostimulation amplitude parameter, and then outputs a corrected amplitude of electrostimulation pulse.

Implementation method 2: open loop control mode.

In the implementation method 2, the patient adjusts the transmission power of the in-vitro energy controller 02. The in-vivo nerve electrostimulator 03 switches to a voltage source type of stimulation pulse mode. In this mode, the cycle, frequency, and corresponding electrode configuration of the electrostimulation pulse remain unchanged. However, the amplitude of the direct current voltage output by the rectification circuit 302 is directly loaded onto both ends of the electrode for stimulation. Therefore, the amplitude of the stimulation is controlled by the transmission power of the in-vitro energy controller 02. At this time, the control is an open-loop control, and the amplitude of the stimulation is completely adjusted manually by the patient.

### New Device Debugging

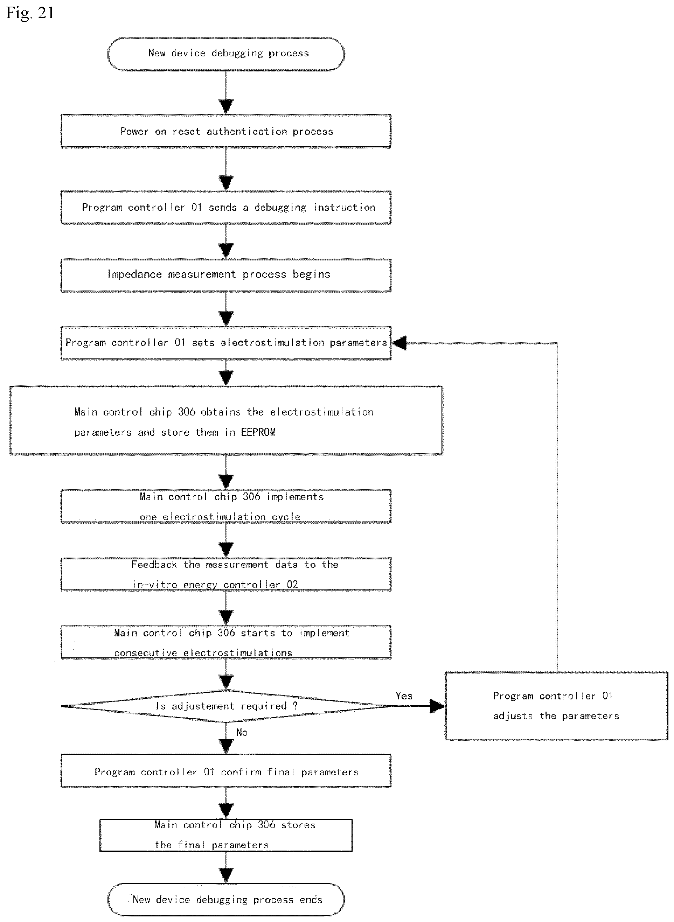

Figure 21 shows a flowchart of debugging a new device.

Controlled by the program controller 01, the program controller 01 sends a debugging instruction to the in-vitro energy controller 02 through Bluetooth. The in-vitro energy controller 02 starts to perform debugging operations, as shown in Figure 21.

The in-vitro energy controller 02 is preferred to start the power on authentication process at first.

After confirming the validity of the chip ID, the in-vitro energy controller 02 transmits a device debugging instruction to the in-vivo nerve electrostimulator 03. After receiving the instruction, the in-vivo nerve electrostimulator 03 begins an impedance measurement program between various electrodes.

After the impedance measurement is completed, the program controller 01 sets the electrostimulation parameters and transmits the parameter information to the in-vivo nerve electrostimulator 03 through the in-vitro energy controller 02. After receiving the electrostimulation parameters, the in-vivo nerve electrostimulator 03 stores all parameters into the internal EEPROM.

After storing the parameters, the main control chip 306 begins the following electrostimulation testing process based on the parameters:
Firstly, the main control chip 306 implements several sets of electrostimulation pulses based on the electrostimulation parameters and completes necessary measurement tasks, namely measuring the inter-electrode voltages. After the stimulation is completed, the measurement data is transmitted to the program controller 01 including an upper computer APP through the in-vitro energy controller 02. Then the electrostimulation pulses continue to be transmitted until next instruction is received or the timer exceeds the limit.

Then, the program controller 01 including an upper computer APP adjusts the stimulation parameters based on the measurement data and patient experience, and performs the above electrostimulation testing process again. This process is repeated until all electrostimulation parameters are confirmed.

Finally, the program controller 01 including an upper computer control APP transmits the finally confirmed parameters to the in-vivo nerve electrostimulator 03 for storage through the in-vitro energy controller 02.

### Abnormal Stimulation Current Processing I

In the conventional implementation process of electrostimulation, a controllable current source circuit is used to generate electrostimulation pulses. If the actually measured current cannot reach the theoretical control value any way, it belongs to an abnormal situation of excessive low current and needs to be dealt with. This is usually caused by factors such as insufficient amplitude of the rectification voltage output by the rectification circuit 302, or changes in electrode impedance.

The main control chip 306 firstly checks the amplitude of the rectification voltage based on the abnormality processing program. If the amplitude of the rectification voltage is insufficient, a request to increase the transmission power is sent to the in-vitro energy controller 02. If the amplitude of the rectification voltage is still insufficient after N (a reasonable value) consecutive requests are sent, a fault code is transmitted and the in-vitro energy controller 02 takes over for processing. If the amplitude of the rectification voltage is normal, or returns to normal after several requests, then the impedance measurement program is activated.

The impedance measurement program is performed and the measured impedance is compared with the originally recorded impedance value. If an abnormality occurs, a fault code is transmitted to the in-vitro energy controller 02. If there is no abnormality, the conventional electrostimulation program is tried again.

If the abnormal stimulation current disappears, the processing is completed. If the abnormal stimulation current still exists after two rounds of the above processing flow, it may be due to a malfunction in the in-vivo electrostimulation itself, then a fault code is transmitted to the in-vitro electrostimulator and the device halts.

### Abnormal Stimulation Current Processing II

During some implementation processes of electrostimulation, abnormality of overhigh current may occur. Due to use of a current source circuit, which has the ability to suppress overhigh current, abnormality of overhigh current usually indicates chip failure. The processing way is to report a fault code to the in-vitro energy controller 02 and the device halts.

### Abnormal Output Voltage of RF Rectification Circuit

If the voltage amplitude is significantly insufficient, please refer to the processing way for insufficient voltage amplitude in the "Patient Manual Adjustment" part. This situation does not fall within the scope of abnormal situations in this item.

If the voltage amplitude is significantly too high, an unknown interference source may appear. At this point, the main control chip 306 sends a fault code and performs short-circuit or impedance switching operation on the antenna, or changes the antenna structure to suppress overhigh voltage to protect the in-vivo nerve electrostimulator 03.

### Charge Balancing Method

This system adopts a combination of active and passive charge balancing ways.

A set of capacitors are connected in series between the interface circuit of the main control chip 306 and the stimulation electrode 307, which are balance capacitors 308. The balance capacitors 308 can ensure that the static charge on each electrode is zero. If the static charge on an electrode is not zero, the main control chip 306 will control the interface circuit to provide a discharge circuit for the balance capacitors 308. The capactor discharging process will automatically achieve charge balance for each electrode.

When the frequency of the electrostimulation pulses is lower, the time period between two electrostimulation pulses is usually longer, which allows the balance capacitor to fully discharge and complete a passive charge balance.

When the frequency of the electrostimulation pulses is higher, the time period between two pulses may not be sufficient for the capacitor to fully discharge, so an active charge balance is required. That is, after implementing an electrostimulation pulse, all configured electrodes are reversed (the emitting electrode becomes the returning electrode, the returning electrode becomes the emitting electrode, and the vacant electrode remains unchanged), and then an electrostimulation pulse having the same amplitude is implemented again. It is equivalent to immediately implementing a same pulse having negative polarity after implementing a positive pulse on the electrode.

The active charge balancing method can also be applied in reverse, that is, implementing a reverse pulse first and then immediately implementing a positive pulse. This implementation way can output an electrostimulation pulse with a higher current amplitude that could not be output in the past.

For convenience of interpretation and precise limitation of the accompanying claims, the terms "up", "down", "inside", and "outside" are used to describe the features of exemplary embodiments by referring to the positions of these features shown in the drawings.
Figure 1
   01 program controller
   02 in-vitro energy controller
   03 in-vivo nerve electrostimulator
   Information
   Electrical energy
Figure 2
   01 program controller
   User input interface
   Bluetooth module
Figure 3
   02 in-vitro energy controller
   202 Bluetooth module
   206 battery module
   203 storage module
   201 energy controller control unit
   204 inverter module
   208 energy controller compensation resonant network
   207 energy controller coupling capacitor pole
   210 WiFi module
   209 button and display module
   205 energy controller harmonic communication module
Figure 4
   03 in-vivo nerve electrostimulator
   301 stimulator coupling capacitor pole
   305 stimulator compensation resonant network
   302 rectification circuit
   304 filter capacitor
   306 main control chip
   303 stimulator harmonic communication module
   308 balance capacitor
   307 stimulation electrode
Figure 5
   306 main control chip
   Bluetooth module
   Voltage stabilizing circuit
   Protection circuit
   Modulation/demodulation circuit
   Controller/memmory
   Controllable current source
   Electrode interface circuit
   Measurement feedback
Figure 6
   Skin
   207 energy controller coupling capacitor pole
   301 stimulator coupling capacitor pole
Figure 7
   204 inverter module
   207 energy controller coupling capacitor pole 207
   301 stimulator coupling capacitor pole
   302 recitification circuit
Figure 8
   204 inverter module
   207 energy controller coupling capacitor pole
   301 stimulator coupling capacitor pole
   302 recitification circuit
Figure 9
   204 inverter module
   207 energy controller coupling capacitor pole
   301 stimulator coupling capacitor pole
   302 recitification circuit
Figure 10
   In-vitro energy controller 02 powers on
   In-vitro energy controller 02 transmits RF carrier wave
   Does the main control chip 306 send a reset signal ?
   Main control chip 306 starts resetting operation
   Main control chip 306 sends chip ID to the in-vitro energy controller 02
   In-vitro energy controller 02 determines whether it is a bonded device
   In-vitro energy controller 02 sends chip ID to the program controller 01
   Program controller 01 confirms that ID is valid and sends a instruction for binding
   In-vitro energy controller 02 stores ID of the main control chip 306 in EEPROM
   In-vitro energy controller 02 sends a verification code to the main control chip 306
   Main control chip 306 receives the verification code and determines whether it is valid
   Main control chip 306 sends an authentication success signal
   Power on authentication process is completed
   Has the authentication failed N times ?
   Authentication failed
Figure 11
   In-vitro energy controller 02 transmits signals
   Voltage of the filter capacitor 304
   Reset signal of the main control chip 306
Figure 12
   Impedance measurement process begins
   In-vitro energy controller 02 sends an impedance measurement request
   Main control chip 306 verifies validity of the request
   Feedback a signal of invalid request
   Impedance measurement program begins
   Measure a voltage output by the rectification circuit 302
   Send data to the in-vitro energy controller 02
   Whether to begin the measurement process ?
   Adjust the inverter module 204
   Output a standard testing pulse to the stimulation electrode 307
   Sample the pulse voltage sequence and store the measurement result in the specified register address through AD conversion
   After completing a standard testing pulse cycle, send the stored voltage pulse sequence to the in-vitro energy controller 02
   The in-vitro energy controller calculates the sequence, completes the algorithm, and obtains the impedance value
   Are all measurement tasks completed ?
   The impedance measurement process is completed
   Replace for the next set of specified stimulation electrodes
Figure 13
   Electrostimulation parameters write in
   Power on reset authentication process
   In-vitro energy controller 02 sends a parameter write-in request
   Main control chip 306 determines validity of the instruction
   In-vitro energy controller 02 sends parameters
   Main control chip 306 stores parameters in the on-chip EEPROM
   Main control chip 306 feedback all writen-in parameters
   In-vitro energy controller 02 receives the signal, and perform verification
   Does it pass verification ?
   Parameters write-in process ends
Figure 14
   Electrostimulation impelmentation process
   Power on reset authentication process
   Main control chip 306 reads electrostimulation parameters from EEPROM into a corresponding register
   Power on reset authentication process
   Measure a voltage output by the rectification circuit 302
   Does the voltage value meets the standard ?
   Configure the selected stimulation electrode pathway
   Set pulse width and cycle counter
   Generate electrostimulation pulse
   Set the stimulation electrode pathway as a charge balancing mode
   Set a counter for the charge balancing process
   Charge balancing process ends, configure all stimulation electrodes 307 to be suspended
   Does the electrostimulation cycle end ?
   Send an instruction to adjust the transmission power
Figure 15
   Voltage of the filter capacitor 304
   Reset signal of the main control chip 306
   Current waveform of electrostimulation pulse
Figure 16
   Current waveform of electrostimulation pulse
Figure 17
   Current waveform of electrostimulation pulse
Figure 18
   Current waveform of electrostimulation pulse
Figure 19
   Current waveform of electrostimulation pulse
Figure 20
   Patient mannual adjustment mode
   Mode 1 / Mode 2
   In-vitro energy controller 02 responds to button
   Send a request to modify the correction cofficient to the main control chip 306
   Main control chip 306 modifies the correction cofficient
   Implement electrostimulation process
   Mode 2
   In-vitro energy controller 02 responds to button
   Send an open-loop control reqest to the main control chip 306
   Main control chip 306 switches to a voltage souce mode
   In-vitro energy controller 02 adjust the transmission power according to button
   Does it reset ?
   Excute the resetting process
   Mannual adjustment mode ends
Figure 21
   New device debugging process
   Power on reset authentication process
   Program controller 01 sends a debugging instruction
   Impedance measurement process begins
   Program controller 01 sets electrostimulation parameters
   Main control chip 306 obtains the electrostimulation parameters and store them in EEPROM
   Main control chip 306 implements one electrostimulation cycle
   Feedback the measurement data to the in-vitro energy controller 02
   Main control chip 306 starts to implement consecutive electrostimulations
   Is adjustement required ?
   Program controller 01 confirm final parameters
   Main control chip 306 stores the final parameters
   New device debugging process ends
   Program controller 01 adjusts the parameters

## Claims

1. An in-vivo nerve electrostimulator (03) comprising:
at least one stimulator coupling capacitor pole (301) for coupling with an energy controller coupling capacitor pole (207) of an in-vitro energy controller (02), thereby receiving electrical energy from the in-vitro energy controller (02) and achieving information exchange;
a stimulator compensation resonant network (305) connected to the stimulator coupling capacitor pole (301) to compensate for reactive power of the received electrical energy;
a rectification circuit (302) connected to the stimulator compensation resonant network (305), and converting alternating current energy that has been compensated by a compensation couple into direct current energy;
a filter capacitor (304) connected to the rectification circuit (302) for direct current filtering the rectified direct current energy;
a main control chip (306) that controls operation of the in-vivo nerve electrostimulator, connected to the rectification circuit (302) and the filter capacitor (304) and receiving power supply therefrom;
a stimulator harmonic communication module (303) connected between the stimulator coupling capacitor pole (301) and the main control chip (306) for modulating and demodulating communication information;
a plurality of sets of stimulation electrodes (307) and corresponding balance capacitors (308), wherein each balance capacitor (308) is connected between the main control chip (306) and a corresponding stimulation electrode (307), receives stimulation pulses from the main control chip (306) and applies them to the corresponding stimulation electrode (307), and achieves charge balance;
wherein the stimulator harmonic communication module (303) comprises a filter circuit sensitive to an A-th harmonic, thereby extracting information from the A-th harmonic of the received signal and transmitting the information to the main control chip (306).

2. The in-vivo nerve electrostimulator (03) according to claim 1, comprising two stimulator coupling capacitor poles (301), wherein the stimulator compensation resonant network (305) is formed as follow: one of the two stimulator coupling capacitor poles (301) is connected to the rectification circuit (302) through a stimulator compensation inductor (L31), and the other one of the two stimulator coupling capacitor poles (301) is directly connected to the rectification circuit (302).

3. The in-vivo nerve electrostimulator (03) according to claim 1, wherein the stimulator harmonic communication module (303) also comprises a filter circuit sensitive to a B-th harmonic, thereby extracting the B-th harmonic from the received signal, and the main control chip (306) controls the stimulator harmonic communication module (303) to modulate impedance of the B-th harmonic, and the impedance modulated data is transmitted to the in-vitro energy controller by the stimulator coupling capacitor pole (301).

4. The in-vivo nerve electrostimulator (03) according to claim 3, wherein B is equal to A.

5. An in-vitro energy controller (02) for transmitting electrical energy to and communicating with an in-vivo nerve electrostimulator, comprising:
a battery module (206) for supplying power to the in-vitro energy controller (02);
an energy controller control unit (201) for controlling the operation of the in-vitro energy controller (02);
an upper computer communication module connected to the energy controller control unit (201) which communicates with a program controller (01) through the upper computer communication module;
a storage module (203) for storing electrical stimulation signals and connected to the energy controller control unit (201);
an inverter module (204) connected to the battery module (206) and the energy controller control unit (201) for converting direct current supplied by the battery module (206) into alternating current;
an energy controller compensation resonant network (208) connected to the inverter module (204) to compensate for reactive power of the system,
at least one energy controller coupling capacitor pole (207) connected to an output end of the energy controller compensation resonant network (208);
an energy controller harmonic communication module (205) powered by the battery module (206) and bridged between the energy controller control unit (201) and the energy controller coupling capacitor pole (207) for modulating and demodulating communication information; and
a button and display module (209) comprising a button for inputting an operation instruction and a display screen for displaying input content and information about the operation of the in-vivo nerve electrostimulator;
wherein the energy controller control unit (201) controls the energy controller harmonic communication module (205) to generate an A-th harmonic equivalent to A times of the output frequency of the inverter module (204), and modulates information into the A-th harmonic.

6. The in-vitro energy controller (02) according to claim 5, comprising two energy controller coupling capacitor poles (207), wherein the energy controller compensation resonant network (208) forms a serial inductance compensation network as follow: one of the two energy controller coupling capacitor poles (207) is connected to the inverter module (204) through a first energy controller compensation inductor (L21), and the other one of the two energy controller coupling capacitor poles (207) is directly connected to the inverter module (204).

7. The in-vitro energy controller (02) according to claim 5, comprising two energy controller coupling capacitor poles (207), wherein the energy controller compensation resonant network (208) forms an LCL compensation network topology as follow: one of the two energy controller coupling capacitor poles (207) is connected in series to the inverter module (204) through one first energy controller compensation inductor (L21) and another first energy controller compensation inductor (L22); the other one of the two energy controller coupling capacitor poles (207) is directly connected to the inverter module (204) to form a connection point, which is connected to a common connection end of the first energy controller compensation inductor (L21) and the first energy controller compensation inductor (L22) through a first energy controller compensation capacitor (C1).

8. The in-vitro energy controller (02) according to claim 5, comprising two energy controller coupling capacitor poles (207), wherein the energy controller compensation resonant network (208) forms a compensation resonant network structure having a relay coil as follow: comprising a primary winding having one end connected to a first output end of the inverter module (204) and another end connected to a second output end of the inverter module (204) through a second energy controller compensation capacitor (C2); and a secondary winding having one end connected to one of the energy controller coupling capacitor poles (207) and another end connected to the other one of the energy controller coupling capacitor poles (207).

9. The in-vitro energy controller (02) according to claim 5, wherein the energy controller control unit (201) controls the energy controller harmonic communication module (205) to generate a B-th harmonic equivalent to B times of the output frequency of the inverter module (204), and transmits the harmonic to the capacitor poles (207).

10. The in-vitro energy controller (02) according to claim 9, wherein B is equal to A.

11. The in-vitro energy controller (02) according to claim 5, wherein the upper computer communication module is a Bluetooth module (202) or a WiFi module.

12. A nerve electrostimulation system using capacitive coupling energy transmission, comprising the in-vivo nerve electrostimulator (03) according to any one of claims 1 to 4 and the in-vitro energy controller (02) according to any one of claims 5 to 11.

13. The nerve electrostimulation system using capacitive coupling energy transmission according to claim 12, further comprising a program controller (01) having a program controller communication module for communicating with the in-vitro energy controller (02) and an upper computer control APP for achieving human-machine interaction control to the in-vitro energy controller (02) and the in-vivo nerve electrostimulator (03).

14. The nerve electrostimulation system using capacitive coupling energy transmission according to claim 13, wherein the program controller communication module is a Bluetooth module or a WiFi module.

## Patentansprüche

1. In-vivo-Nervenelektrostimulator (03), der Folgendes umfasst:
wenigstens einen Stimulatorkopplungskondensatorpol (301) zum Koppeln mit einem Energiesteuereinheits-Kopplungskondensatorpol (207) einer In-vitro-Energiesteuereinheit (02), wodurch elektrische Energie von der In-vitro-Energiesteuereinheit (02) empfangen wird und ein Informationsaustausch erreicht wird;
ein Stimulatorkompensationsresonanznetzwerk (305), das mit dem Stimulatorkopplungskondensatorpol (301) verbunden ist, um die Blindleistung der empfangenen elektrischen Energie zu kompensieren;
eine Gleichrichterschaltung (302), die mit dem Stimulatorkompensationsresonanznetzwerk (305) verbunden ist und die Wechselstromenergie, die durch ein Kompensationspaar kompensiert worden ist, in Gleichstromenergie umsetzt;
einen Filterkondensator (304), der mit der Gleichrichterschaltung (302) für die Gleichstromfilterung der gleichgerichteten Gleichstromenergie verbunden ist;
einen Hauptsteuerchip (306), der den Betrieb des In-vivo-Nervenelektrostimulators steuert, der mit der Gleichrichterschaltung (302) und dem Filterkondensator (304) verbunden ist und hiervon eine Leistungsversorgung empfängt;
ein Stimulatoroberschwingungskommunikationsmodul (303), das zwischen den Stimulatorkopplungskondensatorpol (301) und den Hauptsteuerchip (306) zum Modulieren und Demodulieren von Kommunikationsinformationen geschaltet ist;
mehrere Sätze von Stimulationselektroden (307) und entsprechenden Ausgleichskondensatoren (308), wobei jeder Ausgleichskondensator (308) zwischen den Hauptsteuerchip (306) und eine entsprechende Stimulationselektrode (307) geschaltet ist, Stimulationsimpulse von dem Hauptsteuerchip (306) empfängt und sie an die entsprechende Stimulationselektrode (307) anlegt und einen Ladungsausgleich erreicht;
wobei das Stimulatoroberschwingungskommunikationsmodul (303) eine Filterschaltung umfasst, die gegenüber einer A-ten Oberschwingung empfindlich ist, wodurch Informationen aus der A-ten Oberschwingung des empfangenen Signals extrahiert werden und die Informationen zu dem Hauptsteuerchip (306) übertragen werden.

2. In-vivo-Nervenelektrostimulator (03) nach Anspruch 1, der zwei Stimulatorkopplungskondensatorpole (301) umfasst, wobei das Stimulatorkompensationsresonanznetzwerk (305) wie folgt gebildet ist: einer der beiden Stimulatorkopplungskondensatorpole (301) ist über eine Stimulatorkompensationsinduktivität (L31) mit der Gleichrichterschaltung (302) verbunden, während der andere der beiden Stimulatorkopplungskondensatorpole (301) direkt mit der Gleichrichterschaltung (302) verbunden ist.

3. In-vivo-Nervenelektrostimulator (03) nach Anspruch 1, wobei das Stimulatoroberschwingungskommunikationsmodul (303) außerdem eine Filterschaltung umfasst, die für eine B-te Oberschwingung empfindlich ist, wodurch die B-te Oberschwingung aus dem empfangenen Signal extrahiert wird, und der Hauptsteuerchip (306) das Stimulatoroberschwingungskommunikationsmodul (303) steuert, um die Impedanz der B-ten Oberschwingung zu modulieren, wobei die impedanzmodulierten Daten durch den Stimulatorkopplungskondensatorpol (301) zu der In-vitro-Energiesteuereinheit übertragen werden.

4. In-vivo-Nervenelektrostimulator (03) nach Anspruch 3, wobei B gleich A ist

5. In-vitro-Energiesteuereinheit (02) zum Übertragen elektrischer Energie zu einem und zum Kommunizieren mit einem In-vivo-Nervenelektrostimulator, die Folgendes umfasst:
ein Batteriemodul (206) zum Zuführen von Leistung zu der In-vitro-Energiesteuereinheit (02);
eine Energiesteuereinheits-Steuereinheit (201) zum Steuern des Betriebs der In-vitro-Energiesteuereinheit (02);
ein oberes Computerkommunikationsmodul, das mit der Energiesteuereinheits-Steuereinheit (201) verbunden ist, die über das obere Computerkommunikationsmodul mit einer Programmsteuereinheit (01) kommuniziert;
ein Speichermodul (203) zum Speichern elektrischer Stimulationssignale, das mit der Energiesteuereinheits-Steuereinheit (201) verbunden ist;
ein Wechselrichtermodul (204), das mit dem Batteriemodul (206) und der Energiesteuereinheits-Steuereinheit (201) verbunden ist, um den durch das Batteriemodul (206) zugeführten Gleichstrom in Wechselstrom umzusetzen;
ein Energiesteuereinheits-Kompensationsresonanznetzwerk (208), das mit dem Wechselrichtermodul (204) verbunden ist, um die Blindleistung des Systems zu kompensieren,
wenigstens einen Energiesteuereinheits-Kopplungskondensatorpol (207), der mit einem Ausgangsende des Energiesteuereinheits-Kompensationsresonanznetzwerks (208) verbunden ist;
ein Energiesteuereinheits-Oberschwingungskommunikationsmodul (205), das durch das Batteriemodul (206) mit Leistung versorgt wird und zwischen der Energiesteuereinheits-Steuereinheit (201) und dem Energiesteuereinheits-Kopplungskondensatorpol (207) zum Modulieren und Demodulieren von Kommunikationsinformationen überbrückt; und
ein Tasten- und Anzeigemodul (209), das eine Taste zum Eingeben einer Betriebsanweisung und einen Anzeigeschirm zum Anzeigen von Eingabeinhalt und Informationen über den Betrieb des In-vivo-Nervenelektrostimulators umfasst;
wobei die Energiesteuereinheits-Steuereinheit (201) das Energiesteuereinheits-Oberschwingungskommunikationsmodul (205) steuert, um eine A-te Oberschwingung zu erzeugen, die zum A-Fachen der Ausgangsfrequenz des Wechselrichtermoduls (204) äquivalent ist, und Informationen in die A-te Oberschwingung moduliert.

6. In-vitro-Energiesteuereinheit (02) nach Anspruch 5, die zwei Energiesteuereinheits-Kopplungskondensatorpole (207) umfasst, wobei das Energiesteuereinheits-Kompensationsresonanznetzwerk (208) wie folgt ein serielles Induktivitätskompensationsnetzwerk bildet: einer der beiden Energiesteuereinheits-Kopplungskondensatorpole (207) ist über eine erste Energiesteuereinheits-Kompensationsinduktivität (L21) mit dem Wechselrichtermodul (204) verbunden, während der andere der beiden Energiesteuereinheits-Kopplungskondensatorpole (207) direkt mit dem Wechselrichtermodul (204) verbunden ist.

7. In-vitro-Energiesteuereinheit (02) nach Anspruch 5, die zwei Energiesteuereinheits-Kopplungskondensatorpole (207) umfasst, wobei das Energiesteuereinheits-Kompensationsresonanznetzwerk (208) eine LCL-Kompensationsnetzwerktopologie wie folgt bildet: einer der beiden Energiesteuereinheits-Kopplungskondensatorpole (207) ist durch eine erste Energiesteuereinheits-Kompensationsinduktivität (L21) und eine weitere erste Energiesteuereinheits-Kompensationsinduktivität (L22) in Reihe mit dem Wechselrichtermodul (204) geschaltet; der andere der beiden Energiesteuereinheits-Kopplungskondensatorpole (207) ist direkt mit dem Wechselrichtermodul (204) verbunden, um einen Verbindungspunkt zu bilden, der über einen ersten Energiesteuereinheits-Kompensationskondensator (C1) mit einem gemeinsamen Verbindungsende der ersten Energiesteuereinheits-Kompensationsinduktivität (L21) und der ersten Energiesteuereinheits-Kompensationsinduktivität (L22) verbunden ist.

8. In-vitro-Energiesteuereinheit (02) nach Anspruch 5, die zwei Energiesteuereinheits-Kopplungskondensatorpole (207) umfasst, wobei das Energiesteuereinheits-Kompensationsresonanznetzwerk (208) eine Kompensationsresonanznetzwerkstruktur mit einer Relaisspule wie folgt bildet: sie umfasst eine Primärwicklung, die ein Ende, das mit einem ersten Ausgangsende des Wechselrichtermoduls (204) verbunden ist, und ein weiteres Ende, das über einen zweiten Energiesteuereinheits-Kompensationskondensator (C2) mit einem zweiten Ausgangsende des Wechselrichtermoduls (204) verbunden ist, aufweist; und eine Sekundärwicklung, die ein Ende, das mit einem der Energiesteuereinheits-Kopplungskondensatorpole (207) verbunden ist, und ein weiteres Ende, das mit dem anderen der Energiesteuereinheits-Kopplungskondensatorpole (207) verbunden ist, aufweist.

9. In-vitro-Energiesteuereinheit (02) nach Anspruch 5, wobei die Energiesteuereinheits-Steuereinheit (201) das Energiesteuereinheits-Oberschwingungskommunikationsmodul (205) steuert, um eine B-te Oberschwingung zu erzeugen, die zum B-Fachen der Ausgangsfrequenz des Wechselrichtermoduls (204) äquivalent ist, und die Oberschwingung zu den Kondensatorpolen (207) überträgt.

10. In-vitro-Energiesteuerung (02) nach Anspruch 9, wobei B gleich A ist

11. In-vitro-Energiesteuerung (02) nach Anspruch 5, wobei das obere Computerkommunikationsmodul ein Bluetooth-Modul (202) oder ein WiFi-Modul ist.

12. Nervenelektrostimulationssystem unter Verwendung einer Energieübertragung über kapazitive Kopplung, das den In-vivo-Nervenelektrostimulator (03) nach einem der Ansprüche 1 bis 4 und die In-vitro-Energiesteuereinheit (02) nach einem der Ansprüche 5 bis 11 umfasst.

13. Nervenelektrostimulationssystem unter Verwendung einer Energieübertragung über kapazitive Kopplung nach Anspruch 12, die ferner eine Programmsteuereinheit (01) mit einem Programmsteuereinheits-Kommunikationsmodul zum Kommunizieren mit der In-vitro-Energiesteuereinheit (02) und einer oberen Computersteuerungs-APP zum Erzielen einer Mensch-Maschine-Interaktionssteuerung für die In-vitro-Energiesteuerung (02) und den in-vivo-Nervenelektrostimulator (03) umfasst.

14. Nervenelektrostimulationssystem unter Verwendung einer Energieübertragung über kapazitive Kopplung nach Anspruch 13, wobei das Programmsteuereinheits-Kommunikationsmodul ein Bluetooth-Modul oder ein WiFi-Modul ist.

## Revendications

1. Électrostimulateur nerveux in vivo (03) comprenant :
au moins un pôle de condensateur de couplage de stimulateur (301) pour un couplage avec un pôle de condensateur de couplage de régulateur d'énergie (207) d'un régulateur d'énergie in vitro (02), recevant de ce fait une énergie électrique en provenance du régulateur d'énergie in vitro (02) et obtenant un échange d'informations ;
un réseau résonant de compensation de stimulateur (305) connecté au pôle de condensateur de couplage de stimulateur (301) pour compenser la puissance réactive de l'énergie électrique reçue ;
un circuit de redressement (302) connecté au réseau résonant de compensation de stimulateur (305), et convertissant l'énergie de courant alternatif qui a été compensée par un couple de compensation en énergie de courant continu ;
un condensateur de filtre (304) connecté au circuit de redressement (302) pour filtrer en courant continu l'énergie de courant continu redressée ;
une puce de commande principale (306) qui commande le fonctionnement de l'électrostimulateur nerveux in vivo, connectée au circuit de redressement (302) et au condensateur de filtre (304) et recevant une alimentation électrique ;
un module de communication harmonique de stimulateur (303) connecté entre le pôle de condensateur de couplage de stimulateur (301) et la puce principale de commande (306) pour moduler et démoduler des informations de communication ;
une pluralité d'ensembles d'électrodes de stimulation (307) et de condensateurs d'équilibrage (308) correspondants, chaque condensateur d'équilibrage (308) étant connecté entre la puce de commande principale (306) et une électrode de stimulation (307) correspondante, recevant des impulsions de stimulation en provenance de la puce de commande principale (306) et les appliquant à l'électrode de stimulation (307) correspondante, et obtenant un équilibrage de charge ;
dans lequel le module de communication harmonique de stimulateur (303) comprend un circuit de filtre sensible à une A-ième harmonique, extrayant de ce fait des informations de l'A-ième harmonique du signal reçu et transmettant les informations à la puce de commande principale (306).

2. Électrostimulateur nerveux in vivo (03) selon la revendication 1, comprenant deux pôles de condensateur de couplage de stimulateur (301), dans lequel le réseau résonant de compensation de stimulateur (305) est formé comme suit : l'un des deux pôles de condensateur de couplage de stimulateur (301) est connecté au circuit de redressement (302) par l'intermédiaire d'une inductance de compensation de stimulateur (L31), et l'autre des deux pôles de condensateur de couplage de stimulateur (301) est directement connecté au circuit de redressement (302).

3. Électrostimulateur nerveux in vivo (03) selon la revendication 1, dans lequel le module de communication harmonique de stimulateur (303) comprend également un circuit de filtre sensible à une B-ième harmonique, extrayant de ce fait la B-ième harmonique du signal reçu, et la puce de commande principale (306) commande le module de communication harmonique de stimulateur (303) pour moduler l'impédance de la B-ième harmonique, et les données modulées d'impédance sont transmises au régulateur d'énergie in vitro par le pôle de condensateur de couplage de stimulateur (301).

4. Électrostimulateur nerveux in vivo (03) selon la revendication 3, dans lequel B est égal à A.

5. Régulateur d'énergie in vitro (02) pour transmettre une énergie électrique à un électrostimulateur nerveux in vivo et communiquer avec celui-ci, comprenant :
un module de batterie (206) pour alimenter le régulateur d'énergie in vitro (02) ;
une unité de commande de régulateur d'énergie (201) pour commander le fonctionnement du régulateur d'énergie in vitro (02) ;
un module de communication avec l'ordinateur hôte, connecté à l'unité de commande de régulateur d'énergie (201) qui communique avec un programmateur (01) par l'intermédiaire du module de communication avec l'ordinateur hôte ;
un module de stockage (203) pour stocker des signaux de stimulation électrique et connecté à l'unité de commande de régulateur d'énergie (201),
un module d'onduleur (204) connecté au module de batterie (206) et à l'unité de commande de régulateur d'énergie (201) pour convertir le courant continu fourni par le module de batterie (206) en courant alternatif ;
un réseau résonant de compensation de régulateur d'énergie (208) connecté au module d'onduleur (204) pour compenser une puissance réactive du système,
au moins un pôle de condensateur de couplage de régulateur d'énergie (207) connecté à une extrémité de sortie du réseau résonant de compensation de régulateur d'énergie (208) ;
un module de communication harmonique de régulateur d'énergie (205) alimenté par le module de batterie (206) et ponté entre l'unité de commande de régulateur d'énergie (201) et le pôle de condensateur de couplage de régulateur d'énergie (207) pour moduler et démoduler des informations de communication ; et
un bouton et un module d'affichage (209) comprenant un bouton pour saisir une instruction de fonctionnement et un écran d'affichage pour afficher un contenu d'entrée et des informations sur le fonctionnement de l'électrostimulateur nerveux in vivo ;
dans lequel l'unité de commande de régulateur d'énergie (201) commande le module de communication harmonique de régulateur d'énergie (205) pour générer une A-ième harmonique équivalente à A fois la fréquence de sortie du module d'onduleur (204), et module les informations dans l'A-ième harmonique.

6. Régulateur d'énergie in vitro (02) selon la revendication 5, comprenant deux pôles de condensateur de couplage de régulateur d'énergie (207), dans lequel le réseau résonant de compensation de régulateur d'énergie (208) forme un réseau de compensation d'inductance série comme suit : l'un des deux pôles de condensateur de couplage de régulateur d'énergie (207) est connecté au module d'onduleur (204) par l'intermédiaire d'une première inductance de compensation de régulateur d'énergie (L21), et l'autre des deux pôles de condensateur de couplage de couplage de régulateur d'énergie (207) est directement connecté au module d'onduleur (204).

7. Régulateur d'énergie in vitro (02) selon la revendication 5, comprenant deux pôles de condensateur de couplage de régulateur d'énergie (207), dans lequel le réseau résonant de compensation de régulateur d'énergie (208) forme une topologie de réseau de compensation LCL comme suit : l'un des deux pôles de condensateur de couplage de régulateur d'énergie (207) est connecté en série au module d'onduleur (204) par l'intermédiaire d'une première inductance de compensation de régulateur d'énergie (L21) et d'une autre première inductance de compensation de régulateur d'énergie (L22) ; l'autre des deux pôles de condensateur de couplage de régulateur d'énergie (207) est directement connecté au module onduleur (204) pour former un point de connexion, qui est connecté à une extrémité de connexion commune de la première inductance de compensation de régulateur d'énergie (L21) et de la première inductance de compensation de régulateur d'énergie (L22) par l'intermédiaire d'un premier condensateur de compensation de régulateur d'énergie (C1).

8. Régulateur d'énergie in vitro (02) selon la revendication 5, comprenant deux pôles de condensateur de couplage de régulateur d'énergie (207), dans lequel le réseau résonant de compensation de régulateur d'énergie (208) forme une structure de réseau résonant de compensation ayant une bobine de relais comme suit : comprenant un enroulement primaire ayant une extrémité connectée à une première extrémité de sortie du module d'onduleur (204) et une autre extrémité connectée à une seconde extrémité de sortie du module d'onduleur (204) par l'intermédiaire d'un second condensateur de compensation de régulateur d'énergie (C2) ; et un enroulement secondaire ayant une extrémité connectée à l'un des pôles de condensateur de couplage de régulateur d'énergie (207) et une autre extrémité connectée à l'autre des pôles de condensateur de couplage de régulateur d'énergie (207).

9. Régulateur d'énergie in vitro (02) selon la revendication 5, dans lequel l'unité de commande de régulateur d'énergie (201) commande le module de communication harmonique de régulateur d'énergie (205) pour générer une B-ième harmonique équivalente à B fois la fréquence de sortie du module d'onduleur (204), et transmet l'harmonique aux pôles de condensateur (207).

10. Régulateur d'énergie in vitro (02) selon la revendication 9, dans lequel B est égal à A.

11. Régulateur d'énergie in vitro (02) selon la revendication 5, dans lequel le module de communication avec l'ordinateur hôte est un module Bluetooth (202) ou un module Wi-Fi.

12. Système d'électrostimulation nerveuse utilisant une transmission d'énergie de couplage capacitif, comprenant l'électrostimulateur nerveux in vivo (03) selon l'une quelconque des revendications 1 à 4 et le régulateur d'énergie in vitro (02) selon l'une quelconque des revendications 5 à 11.

13. Système d'électrostimulation nerveuse utilisant une transmission d'énergie de couplage capacitif selon la revendication 12, comprenant en outre un programmateur (01) ayant un module de communication de programmateur pour communiquer avec le régulateur d'énergie in vitro (02) et une application de commande sur l'ordinateur hôte pour réaliser une commande d'interaction homme-machine avec le régulateur d'énergie in vitro (02) et l'électrostimulateur nerveux in vivo (03).

14. Système d'électrostimulation nerveuse utilisant une transmission d'énergie de couplage capacitif selon la revendication 13, dans lequel le module de communication de programmateur est un module Bluetooth ou un module Wi-Fi.
